(19) Europäisches Patentamt
European Patent Office
Office européen des brevets

(11) Veröffentlichungsnummer: **0 174 584**
**A2**

(12) # EUROPÄISCHE PATENTANMELDUNG

(21) Anmeldenummer: **85111079.1**

(22) Anmeldetag: **03.09.85**

(51) Int. Cl.[4]: **C 07 C 103/78**
**A 01 N 37/26**

(30) Priorität: **13.09.84 JP 190605/84**

(43) Veröffentlichungstag der Anmeldung:
**19.03.86 Patentblatt 86/12**

(84) Benannte Vertragsstaaten:
**AT BE CH DE FR GB IT LI NL SE**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Kurahashi, Yoshio**
**47-15, Oya-machi**
**Hachioji-shi Tokyo(JP)**

(72) Erfinder: **Shiokawa, Kozo**
**210-6, Shukugawara Tama-ku**
**Kawasaki-shi Kanagawa-ken(JP)**

(72) Erfinder: **Goto, Toshio**
**3454-21, Honmachida**
**Machida-shi Tokyo(JP)**

(72) Erfinder: **Kagabu, Shinzo**
**432-131-105, Terado-machi**
**Hachioji-shi Tokyo(JP)**

(72) Erfinder: **Matsumoto, Noboru**
**39-15, Namiki-cho**
**Hachioji-shi Tokyo(JP)**

(72) Erfinder: **Moriya, Koichi**
**39-15, Namiki-cho**
**Hachioji-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Salicylamid-Derivate.**

(57) Beschrieben werden neue Salicylamid-Derivate der Formel (I)

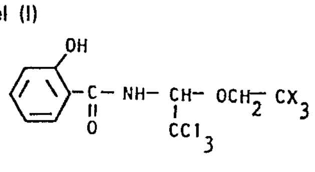

(I)

in der
X ein Halogen-Atom bezeichnet,
und die Verwendung der neuen Verbindungen als Fungizide.

EP 0 174 584 A2

Bag/Th-c

Salicylamid-Derivate

Die vorliegende Erfindung betrifft neue Salicylamid-Derivate, ein Verfahren zu ihrer Herstellung und ihre Verwendung als Fungizide, insbesondere gegen Erkrankungen unterirdischer Pflanzenteile.

Es wurde bereits offenbart, daß ein bestimmtes Salicylamid-Derivat eine Wirksamkeit als Boden-Fungizid besitzt (vgl. die JP-Patentveröffentlichung Nr. 35662/1981).

Nunmehr wurden neue Salicylamid-Derivate der Formel (I) gefunden

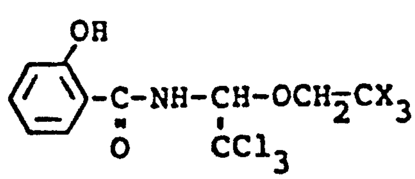

(I),

in der

X Halogen bezeichnet.

Salicylamid-Derivate der Formel (I) werden erhalten, wenn die Verbindungen der Formel (II)

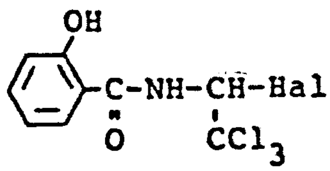

(II),

in der

Hal ein Halogen-Atom bezeichnet,

mit den Verbindungen der Formel (III)

$CX_3CH_2OH$ (III),

in der

X ein Halogen-Atom bezeichnet,

gegebenenfalls in Gegenwart von Säure-Akzeptoren und inerten Lösungsmitteln umgesetzt werden.

Die neuen Salicylamid-Derivate zeigen leistungsfähige fungizide Eigenschaften, insbesondere gegen Pflanzenerkrankungen im Boden.

Überraschenderweise zeigen die Salicylamid-Derivate gemäß der vorliegenden Erfindung eine wesentlich stärkere fungizide Wirkung vor allem gegen Erkrankungen unterirdischer Pflanzenteile als diejenigen, die aus dem Stand der Technik bekannt sind, und insbesondere zeigen die Verbindungen der Formel (I) eine außerordentlich hervorragende Wirksamkeit gegen Hernie (Kropfkrankheit) von Kreuzblütlern.

Unter den Salicylamid-Derivaten gemäß der vorliegenden Erfindung mit der Formel (I) sind bevorzugte Verbindungen solche, in denen

X    Chlor, Fluor oder Brom bezeichnet.

Ganz besonders bevorzugte Salicylamid-Derivate der Formel (I) sind diejenigen, in denen

X    Chlor oder Fluor bezeichnet.

Speziell erwähnt seien die folgenden Verbindungen:
N-[2,2,2-Trichloro-1-(2,2,2-trichloroethoxy)ethyl]-salicylamid;
N-[2,2,2-Trichloro-1-(2,2,2-trifluoroethoxy)ethyl]-salicylamid.

Wenn beispielsweise N-(1,2,2,2-Tetrachloroethyl)-salicylamid und 2,2,2-Trifluoroethanol als Ausgangsstoffe eingesetzt werden, läßt sich der Reaktionsablauf durch die nachstehende Gleichung darstellen:

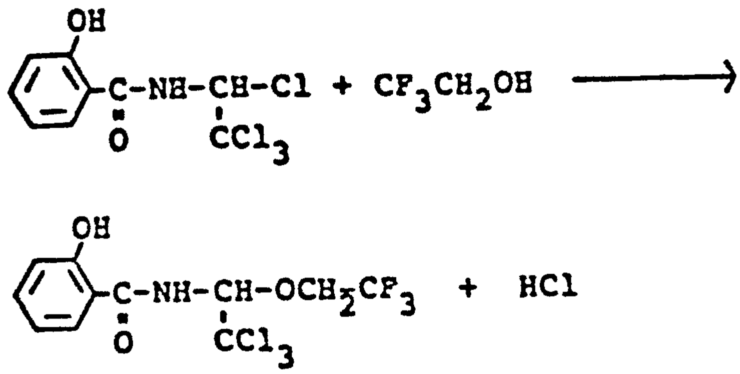

Die Formel (II) liefert eine allgemeine Definition der Verbindungen, die als Ausgangsstoffe in der vorstehenden Reaktionsvariante gemäß der vorliegenden Erfindung benötigt werden.

In der Formel (II) bezeichnet Hal ein Halogen-Atom, vorzugsweise Chlor oder Brom.

Die gemäß der vorliegenden Erfindung einsetzbaren Verbindungen der Formel (II) sind bereits bekannt (vgl. die JP-Patentveröffentlichung Nr. 35662/1981) und können in einfacher Weise durch Umsetzung von Salicylamid mit Chloral und anschließende Reaktion des Produkts mit einem Halogenierungsmittel hergestellt werden.

Als Beispiele seien erwähnt:
N-(1,2,2,2-Tetrachloroethyl)salicylamid:
N-(1-Bromo-2,2,2-trichloroethyl)salicylamid.

Die Formel (III) liefert eine allgemeine Definition der Verbindungen, die als Ausgangsstoffe in der vorstehenden Reaktionsvariante gemäß der vorliegenden Erfindung benötigt werden.

In der Formel (III) hat X vorzugsweise die bereits im Vorstehenden angegebenen Bedeutungen.

Die gemäß der vorliegenden Erfindung einsetzbaren Verbindungen (III) sind bereits bekannt.

Als Beispiele seien erwähnt:
2,2,2-Trifluoroethanol:
2,2,2-Trichloroethanol:
2,2,2-Tribromoethanol.

Geeignete Lösungsmittel sind sämtliche inerten organischen Lösungsmittel. Zu diesen zählen vorzugsweise aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Ester wie Ethylacetat und Amylacetat, Säureamide wie Dimethylformamid und Dimethylacetamidamid, Sulfone und Sulfoxide wie Dimethylsulfoxid und Sulfolan sowie Basen wie Pyridin.

Die obige Reaktion kann in Gegenwart von Säure-Akzeptoren durchgeführt werden. Beispiele für die säurebindenden Mittel umfassen die Hydroxide, Carbonate, Hydrogencarbonate und Alkoholate von Alkalimetallen sowie tertiäre Amine wie Triethylamin, Diethylanilin und Pyridin, die allgemein verwendet werden.

Das obige Verfahren kann in einem weiten Temperaturbereich durchgeführt werden. Im allgemeinen kann es bei einer Temperatur zwischen etwa -20°C und dem Siedepunkt der Mischung, vorzugsweise zwischen etwa 0°C und etwa 100°C, durchgeführt werden. Die Reaktion wird zweckmäßiger unter Normaldruck durchgeführt, jedoch ist es auch möglich, unter erhöhtem oder vermindertem Druck zu arbeiten.

Die aktiven Substanzen gemäß der vorliegenden Erfindung zeigen leistungsfähige vor allem protektive Wirkungen gegenüber Pflanzenerkrankungen im Boden. Aus diesem Grunde können sie als Fungizide vor allem gegen Erkrankungen unterirdischer Pflanzenteile eingesetzt werden.

Speziell zeigen die aktiven Substanzen eine außerordentlich herausragende protektive Wirkung gegenüber der Hernie (Kropfkrankheit; Plasmodiophora brassicae) von Kreuzblütern.

Die Verbindungen gemäß der vorliegenden Erfindung zeigen auch eine überlegene Wirksamkeit gegen die durch Aphanomyces cochlioides verursachte Erkrankung, eine Art Umfallen (Wurzelbrand) von Reis-Setzlingen, und besitzen

überdies ausgezeichnet fungizide Aktivität gegen Mikroorganismen, die mit Hilfe industrieller Fungizide breit bekämpft werden sollen, beispielsweise Aerobacter aerogenes, Staphylococcus aureus, Bacillus subtilis, Escherichia coli, Aspergillus niger, Trichoderma viride, Penicillium sp., Rhizopus sp. und Fusarium sp..

Als Fungizide gegen Pflanzenerkrankungen im Boden, beispielsweise zur Verhütung der Hernie von Kreuzblütlern, können die Verbindungen der Formel (I) gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese Präparate entweder unmittelbar oder nach dem Verdünnen mit Wasser auf die gewünschte Konzentration zur Anwendung gebracht.

Die landwirtschaftlich unbedenklichen Hilfsstoffe, auf die hier Bezug genommen wird, umfassen beispielsweise Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Lösungsmittel können Wasser und organische Lösungsmittel sein. Beispiele für die organischen Lösungsmittel sind Kohlenwasserstoffe [z.B. n-Hexan, Petrolether, Erdöl-

Fraktionen (z.B. Paraffinwachse, Kerosin, Leichtöle, Mittelöle und Schweröle), Benzol, Toluol und Xylol], halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methanol, Ethanol, Propanol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat) Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Koalinit und Glimmer), pflanzliche Pulver wie Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (z.B. Natriumlaurylsulfat), Arylsulfonsäuren (z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin.

Stearyltrimethylammoniumchlorid und Alkyldimethylbenzylammoniumchlorid) und Polyoxyethylenalkylamine, nichtionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), wirkungsverlängernde Mittel, Dispersions-Stabilisatoren [z.B. Casein, Tragant, Carboxymethylcellulose (CMC) und Polyvinylalkohol (PVA)] und synergistische Mittel.

Die Verbindungen gemäß der vorliegenden Erfindung können mittels allgemein bei der Herstellung von Agrochemikalien angewandter Methoden zu verschiedenartigen Präparaten formuliert werden. Erläuternde Beispiele für solche Anwendungsformen sind emulgierbare Konzentrate, benetzbare Pulver, lösliche Pulver, Suspensionen, Stäubemittel, Granulat, Pulverpräparate und Kapseln. Stäubemittel, Granulat und Pulverpräparate werden bevorzugt.

Das Mittel zur Bekämpfung der Pilze gemäß der vorliegenden Erfindung kann etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Art des Präparats, dem Verfahren, Zweck, der Zeit und dem Ort seiner Anwendung, dem Zustand des Auftretens der Krankheit etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen [Organophosphat-Verbindungen, Carbamat-Verbindungen, Dithio-(oder thiol)carbamat-Verbindungen, organischen Chlor-Verbindungen, Dinitro-Verbindungen, organischen Schwefel- oder Metall-Verbindungen, Antibiotika, substituierten Diphenyleter-Verbindungen, Harnstoff-Verbindungen und Triazin-Verbindungen] und/oder Düngemitteln.

Die den im Vorstehenden bezeichneten Wirkstoff enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Bodenbehandlung (Vermischen, Streuen, Bedampfen und Gießen etc.). Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,5 bis etwa 30 kg/ha, vorzugsweise etwa 1 bis etwa 20 kg/ha. In besonders gelagerten Fällen können oder sollten sogar die Aufwandmengen außerhalb des angegebenen Bereichs liegen.

Gemäß der vorliegenden Erfindung kann vor allem eine Zusammensetzung zur Bekämpfung der Hernie von Kreuzblütlern verfügbar gemacht werden, die eine Verbindung der allgemeinen Formel (I) als Wirkstoff und ein Verdünnungsmittel (ein Lösungsmittel und/oder ein Streckmittel und/oder einen Träger) und/oder ein oberflächenaktives Mittel und, sofern weiter erforderlich, einen Stabilisator, ein Haftmittel und ein synergistisches Mittel enthält.

Die vorliegende Erfindung stellt ebenfalls ein Verfahren vor allem zur Bekämpfung der Hernie von Kreuzblütlern zur Verfügung, bei dem auf einen Hernie-Pilz und/oder den Ort seines Auftretens eine Verbindung der allgemeinen Formel (I) entweder allein oder im Gemisch mit einem Verdünnungsmittel (einem Lösungsmittel und/oder einem Streckmittel und/oder einem Träger) und/oder einem oberflächenaktiven Mittel und, sofern weiter erforderlich, einem Stabilisator, einem Haftmittel und einem synergistischen Mittel etc. aufgebracht wird.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

**Herstellungsbeispiele:**

**Beispiel 1**

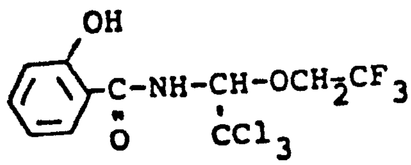

(Verbindung Nr. 1)

Eine Lösung von N-(1,2,2,2-Tetrachloroethyl)salicylamid (30 g) in Toluol (100 ml) wird tropfenweise bei einer Temperatur unterhalb von 10°C zu einer Lösung von 2,2,2-Trifluoroethanol (10 g) und Pyridin (18,6 g) in Toluol (150 ml) hinzugefügt. Nach der Zugabe wird die Mischung eine Weile bei Raumtemperatur gerührt, und der Inhalt wird mit Wasser und 1-proz. Natriumhydrogencarbonat-Lösung gewaschen. Die Toluol-Schicht wird entwässert, und das Toluol wird unter vermindertem Druck abgedampft, wonach das gewünschte N-[2,2,2-Trichloro-1-(2,2,2-trifluoroethoxy)ethyl]salicylamid (26 g) der vorstehenden Formel als farblose viskose Masse erhalten wird. Die Verbindung erstarrt allmählich und zeigt einen Schmelzpunkt von 78-82°C.

Beispiel 2

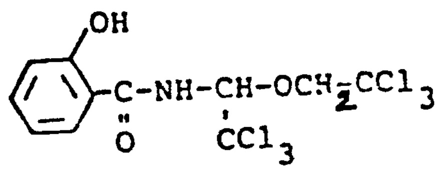

(Verbindung Nr. 2)

Eine Lösung aus 2,2,2-Trichloroethanol (30 g), N-(1,2,2,2-Tetrachloroethyl)salicylamid (30 g) und Toluol (200 ml) wird am Rückfluß erhitzt, bis die Entwicklung von Hydrogenchlorid beendet ist (etwa 5 bis 8 h). Die Reaktionsmischung wird auf Raumtemperatur abgekühlt. Die Toluol-Schicht wird mit Wasser gewaschen und entwässert, und das Toluol wird unter vermindertem Druck abgedampft, wonach das gewünschte N-[2,2,2-Trichloro-1-(2,2,2-trichloroethoxy)ethyl]salicylamid (37 g) der vorstehenden Formel in Form farbloser Kristalle erhalten wird; Schmp. 111-114°C.

Nach der gleichen Arbeitsweise wie vorstehend werden 2,2,2-Tribromoethanol und N-(1,2,2,2-Tetrachloroethyl)-salicylamid umgesetzt, wodurch N-[2,2,2-Trichloro-1-(2,2,2-tribromoethoxy)ethyl]salicylamid (Verbindung Nr. 3 der Erfindung) erhalten wird.

Verwendungsbeispiele:

Die bekannten Vergleichs-Verbindungen werden folgendermaßen identifiziert:

Vergleichs-Verbindung A-1:

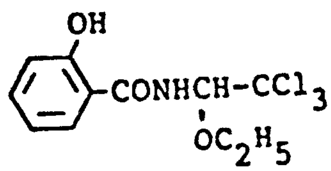

(die in der JP-Patentveröffentlichung Nr. 35662/1981 beschriebene Verbindung)

PCNB: Pentachloronitrobenzol (20 % Stäubemittel)

Beispiel 3

Test der Bekämpfung der Kohlhernie (Kropfkrankheit) von "Komatsuna" (einer chinesischen Kohl-Art):

Test-Verfahren:

Unglasierte Töpfe mit einem Durchmesser von 21 cm werden mit Erde gefüllt, die mit dem Kohlhernie-Pilz (Plasmodiophora brassicae) infiziert ist, und ein Stäubemittel der Verbindung gemäß der vorliegenden Erfindung, das mittels des gleichen Verfahrens wie in Beispiel 5 hergestellt wurde, wird gleichmäßig mit der Erde vermischt, so daß eine vorher festgelegte Konzentration eingestellt wird.

Samen von Komatsuna (Varietät: Misugi) werden in einer Menge von 20 pro Topf auf die Erde gesät. Die Töpfe werden 4 Wochen bei 20°C in einem Gewächshaus unter kontrollierten Belüftungsbedingungen gehalten. Danach werden die Pflanzen ausgegraben, und der Krankheitszustand wird aufgrund des folgenden Maßstabs bewertet. Der Erkrankungs-Index und die Rate erkrankter Pflanzen werden berechnet.

Grad der Erkrankung:

3: $L_1$, die Zahl der Pflanzen, deren Wurzeln infolge des Auftretens des Kropfes in ausgeprägter Weise vergrößert sind;

2: $L_2$, die Zahl der Pflanzen, bei denen das Auftreten des Kropfes beobachtet wird, jedoch auf die Hauptwurzeln beschränkt ist;

1: $L_3$, die Zahl der Pflanzen, bei denen das Auftreten des Kropfes nur in geringem Umfang beobachtet wird;

0: $L_4$, die Zahl der gesunden Pflanzen.

$$\text{Erkrankungs-Index} = \frac{3 \times L_1 + 2 \times L_2 + 1 \times L_3}{3 \times (L_1 + L_2 + L_3 + L_4)} \times 100$$

$$\text{Rate erkrankter Pflanzen} = \frac{L_1 + L_2 + L_3}{L_1 + L_2 + L_3 + L_4} \times 100$$

| Verbindung Nr. | Wirkstoff-Menge (kg/ha) | Erkrankungs-Index (%) | Rate erkrankter Pflenzen (%) | Phyto-Toxizität |
|---|---|---|---|---|
| 1 | 20 | 0 | 0 | keine |
| | 10 | 0 | 0 | " |
| | 5 | 0 | 0 | " |
| 2 | 20 | 0 | 0 | keine |
| | 10 | 0 | 0 | " |
| | 5 | 1,7 | 5 | " |
| Vergleich | | | | |
| A-1 | 20 | 33,3 | 45 | keine |
| | 10 | 80 | 85 | " |
| | 5 | 81,7 | 95 | " |
| PCNB (Handelsprodukt) | 60 | 58,3 | 75 | keine |
| unbenandelt | - | 95 | 100 | " |

Nit 185 -Ausland

Beispiel 4

2 Teile der Verbindung Nr. 1 der Erfindung und 98 Teile Tonpulver werden pulverisiert und gemischt, wodurch ein Stäubemittel hergestellt wird. Dieses wird über dem Boden, in dem das Auftreten der Kohlhernie vermutet wird, ausgestreut und mit diesem vermischt.

Beispiel 5

Die Verbindung Nr. 2 der Erfindung (1,5 Teile), 0,5 Teile Isopropylhydrogenphosphat (PAP) und 98 Teile Tonpulver werden pulverisiert und gemischt, wodurch ein Stäubemittel hergestellt wird. Dieses wird über dem Boden, in dem Kohlhernie auftritt oder ihr Auftreten vermutet wird, ausgestreut und mit diesem vermischt.

Beispiel 6

Wasser (25 Teile) wird zu einer Mischung aus 10 Teilen der Verbindung Nr. 1 der Erfindung, 30 Teilen Bentonit (Montmorillonit), 58 Teilen Talkum und 2 Teilen Lignosulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet, das dann bei 40°C bis 50°C getrocknet wird. Das Granulat wird über dem Boden, in dem Kohlhernie auftritt oder ihr Auftreten vermutet wird, ausgestreut und mit diesem vermischt.

Beispiel 7

Ein Drehmischer wird mit 95 Teilen Tonmineral-Teilchen mit einer Teilchengrößen-Verteilung zwischen 0,2 und 2 mm beschickt, und unter Drehen des Mischers werden 5 Teile der Verbindung Nr. 2 der Erfindung gelöst in einem organischen Lösungsmittel auf die Teilchen zur gleichmäßigen Benetzung derselben aufgesprüht, wodurch ein Granulat gebildet wird. Dieses wird dann bei 40°C bis 50°C getrocknet. Das Granulat wird über dem Boden, in dem Kohlhernie auftritt oder ihr Auftreten vermutet wird, ausgestreut und mit diesem vermischt.

## Patentansprüche

1. Salicylamid-Derivate der Formel (I)

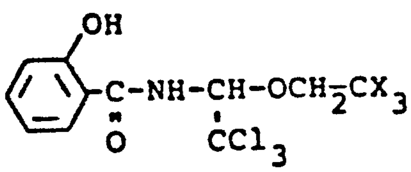

(I),

in der

X ein Halogen-Atom bezeichnet.

2. Verbindungen der Formel (I) nach Anspruch 1, dadurch gekennzeichnet, daß X Chlor, Fluor oder Brom, vorzugsweise Chlor oder Fluor, bezeichnet.

3. Verfahren zur Herstellung von Salicylamid-Derivaten der Formel (I)

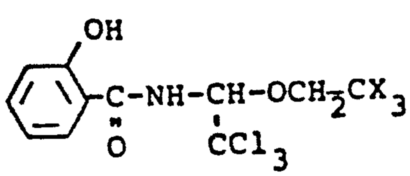

(I),

Nit 185 -Ausland

in der

X ein Halogen-Atom bezeichnet,

dadurch gekennzeichnet, daß die Verbindungen der Formel (II)

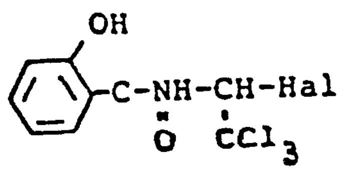

(II),

in der

Hal ein Halogen-Atom bezeichnet,

mit den Verbindungen der Formel (III)

$CX_3CH_2OH$ (III),

in der

X die angegebene Bedeutung hat,

gegebenenfalls in Gegenwart von Säure-Akzeptoren und inerten Lösungsmitteln umgesetzt werden.

4. Fungizide Mittel, dadurch gekennzeichnet, daß sie mindestens ein Salicylamid-Derivat der Formel (I) nach den Ansprüchen 1 bis 3 enthalten.

5. Fungizide Mittel gemäß Anspruch 4 zum Einsatz im Pflanzenschutz.

6. Verfahren zur Bekämpfung von Pilzen, dadurch gekennzeichnet, daß man Salicylamid-Derivate der Formel (I) nach den Ansprüchen 1 bis 3 auf Pilze und/oder ihren Lebensraum einwirken läßt.

7. Verwendung von Salicylamid-Derivaten der Formel (I) nach den Ansprüchen 1 bis 3 zur Bekämpfung von Pilzen.

8. Verfahren zur Herstellung fungizider Mittel, dadurch gekennzeichnet, daß Salicylamid-Derivate der Formel (I) nach den Ansprüchen 1 bis 3 mit Streckmitteln und/oder oberflächenaktiven Mitteln vermischt werden.

9. Verwendung von Salicylamid-Derivaten der Formel (I) nach Anspruch 7 zur Verhütung von Erkrankungen unterirdischer Pflanzenteile, insbesondere der Pflanzenkrankheit Hernie (Kropfkrankheit) der Kreuzblütler.

10. Verfahren zur Herstellung fungizider Zusammensetzungen nach Anspruch 8 gegen Pflanzenkrankheitem im Boden.